# EUROPÄISCHE PATENTSCHRIFT

(11) **EP 1 117 711 B1**
(45) Veröffentlichungstag und Bekanntmachung des Hinweises auf die Patenterteilung: **05.01.2005**
(21) Anmeldenummer: 99945994.4
(22) Anmeldetag: 19.08.1999
(51) Int. Cl.: C08F 220/18, C08F 220/54, A61K 7/06

(54) **KATIONISCHE POLYMERISATE UND IHRE VERWENDUNG**
CATIONIC POLYMERS AND THEIR USE
POLYMERES CATIONIQUES ET LEUR UTILISATION

(30) Priorität: 24.08.1998 DE 19838196
(43) Veröffentlichungstag der Anmeldung: 25.07.2001
(73) Patentinhaber: BASF AKTIENGESELLSCHAFT, 67056 Ludwigshafen (DE)
(72) Erfinder: NGUYEN KIM, Son, D-69502 Hemsbach (DE); SANNER, Axel, D-67227 Frankenthal (DE); SCHEHLMANN, Volker, D-67105 Schifferstadt (DE)
(86) Internationale Anmeldenummer: PCT/EP1999/006059
(87) Internationale Veröffentlichungsnummer: WO 2000/011051

(56) Entgegenhaltungen:
- EP-A- 0 100 890
- EP-A- 0 373 442
- WO-A-96/19966

## Beschreibung

Die vorliegende Erfindung betrifft kationische Polymerisate, erhältlich durch radikalische Copolymerisation von
(a) 50 bis 70 Gew.-% eines oder mehrerer Monomeren der Formel I 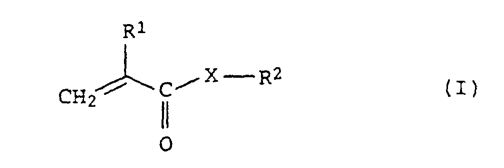
   X = O, NR¹,
   R¹ = H, C₁-C₈-Alkyl,
   R² = tert.-Butyl,
(b) 5 bis 45 Gew.-% eines oder mehrerer Monomeren der Formel II 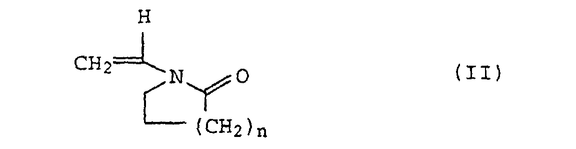 mit n = 1 bis 3,
(c) 5 bis 40 Gew.-% eines monoethylenisch ungesättigten Monomers mit mindestens einer aminhaltigen Gruppe,
(d) 0 bis 5 Gew.-% eines polyalkylenoxid-haltigen Silikonderivats,
wobei bis zu 40 Gew.-%, bezogen auf (a), (b), (c) und (d) des Monomeren (a) durch ein Monomer der Formel I mit R² = C₂-C₂₂-Alkyl ersetzt sein können.

In der Kosmetik werden Polymere mit filmbildenden Eigenschaften zur Festigung, Strukturverbesserung und Formgebung der Haare verwendet. Diese Haarbehandlungsmittel enthalten im allgemeinen eine Lösung des Filmbildners in einem Alkohol oder einem Gemisch auf Alkohol und Wasser.

Haarfestigungsmittel werden im allgemeinen in Form von wäßrigalkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Eine weitere Anforderung an Haarbehandlungsmittel ist, dem Haar ein natürliches Aussehen und Glanz zu verleihen, z.B. auch dann, wenn es sich um von Natur aus besonders kräftiges und/oder dunkles Haar handelt.

Ein Nachteil vieler bekannter Haarfestigerpolymere ist der sogenannte "Flaking"-Effekt, d.h. nach dem Auskämmen bleibt ein weißer, schuppenförmiger Rest auf dem Haar zurück. Dies wird von den Anwendern im allgemeinen als äußerst unangenehm empfunden. Der "Flaking"-Effekt tritt besonders deutlich bei Personen mit dunkler Haarfarbe und/oder besonders kräftigen Haaren auf. Die Einsatzmöglichkeit von Haarfestigerformulierungen, die diesen Effekt aufweisen, ist somit insbesondere auf dem asiatischen Markt deutlich beeinträchtigt. Als mögliche Ursache für den "Flaking"-Effekt werden u.a. die chemische Struktur der eingesetzten Haarfestigerpolymere und insbesondere die Partikelgröße des Sprays angesehen.

Neben den zuvor genannten Eigenschaften sollen Haarfestigerpolymere daher vorzugsweise eine hohe Treibgasverträglichkeit aufweisen, um eine Formulierung in Spraydosen unter möglichst hohem Druck zuzulassen. Dies gilt sowohl für die klassischen Treibmittel auf Propan/Butan-Basis als auch für deren Ersatzstoffe, z.B. auf Dimethyletherbasis.

Kationische Haarfestigerpolymere sind beispielsweise aus US 3,914,403 und 3,954,960 und US 4,057,533 bekannt.

Diese Polymere bestehen aus Vinylpyrrolidon, einem quaternierbaren Monomer, beispielsweise Dimethylaminoethylmethacrylat (DMAEMA) und gegebenenfalls einem weiteren Monomer.

WO 90/01920, WO 96/19966 und WO 96/20694 beschreiben Polymere aus Vinylpyrrolidon, einem quaternierbaren Monomer und bis zu 49 % eines weiteren hydrophoben Polymers.

Die Verträglichkeit dieser Polymere mit dem Treibmittel Propan/Butan ist jedoch nicht für alle gewünschten Anwendungen ausreichend. Außerdem sind die Produkte aufgrund des hohen VP-Anteils sehr klebrig und verlieren bei hoher Luftfeuchtigkeit an Festigungswirkung.

Anionische Polymere mit Propan/Butan-Verträglichkeit sind bereits bekannt, unter anderem Polymere auf Basis von tert.-Butylacrylat bzw. tert.-Butylmethacrylat.

Die EP-A-379 082 beschreibt z.B. ein Haarfestigungsmittel, enthalten als Filmbildner ein Copolymerisat, welches
A) 75 bis 99 Gew.-% tert.-Butyl(meth)acrylat,
B) 1 bis 25 Gew.-% (Meth)acrylsäure und
C) 0 bis 10 Gew.-% eines weiteren radikalisch copolymerisierbaren hydrophoben Monomeren
einpolymerisiert enthält. Haarfestigungsmittel auf Basis dieser Copolymere, die nur die Komponenten A) und B) enthalten, machen das Haar zu hart und weisen eine zu geringe Propan/Butan-Verträglichkeit auf. Copolymere, die zusätzlich ein Monomer C) enthalten, sind bezüglich ihrer Auswaschbarkeit verbesserungswürdig.

Die DE-A-43 14 305 beschreibt wie die EP-A-379 082 ein Haarfestigerpolymer auf Basis von tert.-Butyl(meth)acrylat und (Meth)acrylsäure, welches 0 bis 60 Gew.-% eines C₁- bis C₁₈-Alkyl(meth)acrylats oder einer Mischung davon mit N-C₁- bis - C₁₈-Alkyl(meth)acrylamiden einpolymerisiert enthält. Zusätzliche Monomere mit einer Kohlenstoffzahl von mehr als 8 führen zwar unter Umständen zu einer besseren Propan/Butan-Verträglichkeit, wobei jedoch gleichzeitig die Auswaschbarkeit deutlich verschlechtert wird.

Der vorliegenden Erfindung liegt die Aufgabe zugrunde, neue kosmetische Mittel, insbesondere Haarbehandlungsmittel, zur Verfügung zu stellen, die neben den guten festigenden Eigenschaften eine hohe Treibgasverträglichkeit aufweisen und im wesentlichen keinen "Flaking"-Effekt zeigen. Vorzugsweise sollen diese Mittel dem Haar Glätte und Geschmeidigkeit verleihen.

Überraschenderweise wurde nun gefunden, daß diese Aufgabe durch kosmetische Mittel gelöst wird, die wenigstens ein wasserlösliches oder wasserdispergierbares kationisches Polymer enthalten, das erhältlich ist durch radikalische Polymerisation von
(a) 50 bis 70 Gew.-% eines oder mehrerer Monomeren der Formel I 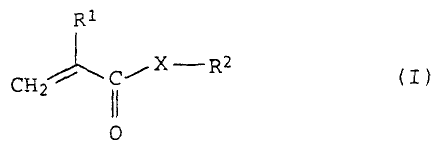
   X = O, NR¹,
   R¹ = H, C₁-C₈-Alkyl,
   R² = tert.-Butyl,
(b) 5 bis 45 Gew.-% eines oder mehrerer Monomeren der Formel II 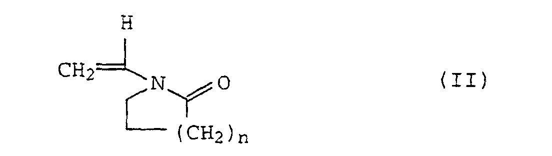 mit n = 1 bis 3,
(c) 5 bis 40 Gew.-% eines monoethylenisch ungesättigten Monomers mit mindestens einer aminhaltigen Gruppe,
(d) 0 bis 5 Gew.-% eines polyalkylenoxid-haltigen Silikonderivats,
wobei bis zu 40 Gew.-%, bezogen auf (a), (b), (c) und (d) des Monomeren (a) durch ein Monomer der Formel I mit R² = C₂-C₂₂-Alkyl ersetzt sein können.

Als bevorzugte Monomere (a) kommen Verbindungen der Formel I in Betracht, in der R¹ H und CH₃ und X O und NH bedeuten; besonders bevorzugt sind tert.-Butylacrylat, N-tert.-Butylacrylamid und/oder tert.-Butylmethacrylat.

Monomer I kann auch aus Mischungen mit unterschiedlicher Bedeutung von R¹ und X bestehen, wobei bevorzugt die Monomeren (a) mit der Bedeutung X = NR¹ nur in Mengen bis zu 20 Gew.-% bezogen auf (a), (b) und (c) eingesetzt werden.

Das Monomer (a) wird bevorzugt in Mengen von 51 bis 65 Gew.-%, Monomer (b) bevorzugt in Mengen von 7 bis 39 Gew.-% und Monomer (c) bevorzugt in Mengen von 10 bis 30 Gew.-% eingesetzt.

Im Rahmen der vorliegenden Erfindung umfaßt der Ausdruck "C₂-C₂₂-Alkyl" geradkettige, verzweigte und cyclische Alkylgruppen. Vorzugsweise handelt es sich dabei um geradkettige und insbesonders um verzweigte Alkylgruppen.

Als Monomer der Formel I, in der R² = C₂-C₂₂-Alkyl bedeutet, sind die folgenden Verbindungen bevorzugt:
N-Butyl(meth)acrylat, n-Octyl(meth)acrylat, 1,1,3,3-Tetramethylbutyl(meth)acrylat, Ethylhexyl(meth)acrylat, n-Nonyl(meth)-acrylat, n-Decyl(meth)-acrylat, n-Undecyl(meth)acrylat, Tridecyl(meth)acrylat, Myristyl(meth)acrylat, Pentadecyl(meth)-acrylat, Pamityl-(meth)acrylat, Heptadecyl(meth)acrylat, Nonadecyl(meth)acrylat, Arrachinyl(meth)acrylat, Behenyl(meth)-acrylat, Lignocerenyl-(meth)acrylat, Cerotinyl(meth)acrylat, Melissinyl(meth)acrylat, Palmitoeinyl(meth)acrylat, Oleyl(meth)-acrylat, Linolyl(meth)-acrylat, Stearyl(meth)acrylat, Lauryl-(meth)-acrylat und Mischungen davon sowie n-tert.-Butyl(meth)-acrylamid, n-Octyl(meth)acrylamid, 1,1,3,3-Tetramethylbutyl-(meth)acrylamid, Ethylhexyl(meth)acrylamid, n-Nonyl(meth)acrylamid, n-Decyl(meth)acrylamid, n-Undecyl(meth)acrylamid, Tridecyl-(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)-acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Stearyl(meth)acrylamid, Lauryl(meth)acrylamid und Mischung davon.

Ganz besonders bevorzugt sind:
N-Butyl(meth)acrylat, n-Octyl(meth)acrylamid, Laurylacryl(meth)acrylat und Stearyl(meth)acrylat, wobei der Ausdruck "(Meth)acrylat" die Methacrylate und Acrylate einschließt.

Monomere (b) sind Verbindungen der Struktur II 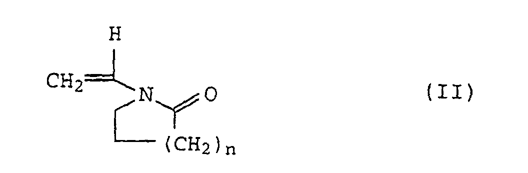 mit n = 1 bis 3,
bevorzugt sind Vinylpyrrolidon (VP), Vinylcaprolactam (VCap) oder Mischungen davon.

Ein Teil von (II), bis zu 4 Gew.-%, bezogen auf die gesamten (a) + (b) + (c), kann durch ein hydrophiles nichtionisches Monomer der Struktur III ersetzt sein 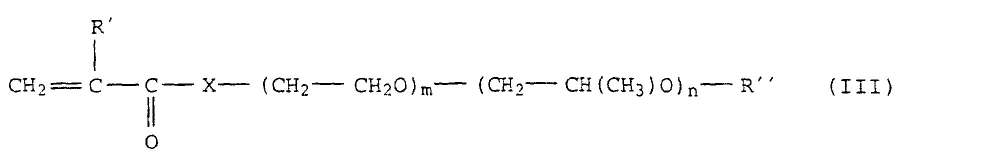
R' = H, CH₃
X = O, NH
m = 0 bis 50
n = 0 bis 50
m + n ≥ 5
R" = H, C₁-C₂₂-Alkyl oder Phenyl.

In der Formel III bedeutet R" bevorzugt ein Wasserstoffatom und eine C₁₋₄-Alkylgruppe, wobei eine Methylgruppe insbesondere bevorzugt ist. Beispiele eines solchen (Meth)acrylat-Monomers umfassen Hydroxy(meth)acrylat, Hydroxypropyl(meth)acrylat, Polyethylenglycolmono (meth) acrylat, Polypropylenglycolmono(meth)acrylat, Methoxypolyethylenglycol(meth)acrylat, Methoxypolypropylenglycolmono (meth) acrylat, Ethoxypolyethylenglycolmono(meth)acrylat, Butoxypolyethylenglycolmono(meth)acrylat und Phenoxypolyethylenglycolmono(meth)acrylat.

Als Monomere (c) werden solche Monomere eingesetzt, die mindestens eine monoethylenisch ungesättigte Gruppe und mindestens eine aminhaltige Gruppe besitzen.

Bevorzugte Monomere (c) sind Verbindungen, dargestellt durch die folgende allgemeine Struktur IV 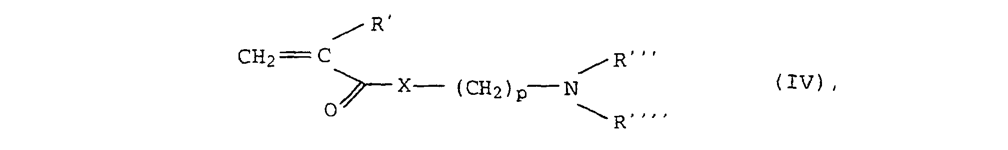 wobei
R' = H, CH₃
X = O, NH
R' ' ' , R' ' ' ' = gleich oder verschieden sind und für CH₃, -C₂H₅, -C₃H₇, -C₄H₉, -tert.-C₄H₉ stehen können
p = 1 bis 5

Besonders geeignete Monomere (c) sind:
- N,N-Dimethylaminoethyl(meth)acrylat
- N,N-Dimethylaminopropyl(meth)acrylat
- N,N-Dimethylaminoethyl(meth)acrylamid
- N,N-Dimethylaminopropyl(meth)acrylamid.

Ganz besonders geeignete Monomere (c) sind:
- N,N-Dimethylaminoethylmethacrylat
- N,N-Dimethylaminoethylmethacrylamid
- N, N-Dimethylaminopropylmethacrylamid.

Die Monomeren (c) werden in Mengen von 5 bis 40, bevorzugt 10 bis 30 Gew.-% eingesetzt.

Geeignete Silikonderivate (d) sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhöne-Poulenc), Silicone Polyol Copolymer® (der Fa Genesee), Belsil® (der Fa. wacker), Silwet® (der Fa. Witco, Greenwich, CT, USA) oder Dow Corning (der Fa. Dow Corning) erhältlich Diese beinhalten Verbindungen mit den CAS-Nummern 64365-23-7; 68937-54-2; 68938-54-5; 68937-55-3.

Die Monomeren der Gruppen (a) bis (d) können jeweils einzeln oder im Gemisch mit weiteren Monomeren der gleichen Gruppe eingesetzt werden.

Die Herstellung der Polymerisate kann nach den an sich bekannten Verfahren der radikalisch initiierten Polymerisation, z.B. durch Lösungspolymerisation, Emulsionspolymerisation, Suspensionspolymerisation, Fällungspolymerisation, Umgekehrte Suspensionspolymerisation oder Umgekehrte Emulsionspolymerisation erfolgen, ohne daß die verwendbaren Methoden darauf beschränkt sind.

Vorzugsweise erfolgt die Herstellung als Lösungspolymerisation in Lösungsmitteln wie Wasser, Methanol, Ethanol, Isopropanol oder Mischungen dieser Lösungsmittel. Man wählt die Mengen an Monomeren und Lösungsmitteln zweckmäßigerweise so, daß 30 bis 70 gew.-%ige Lösungen entstehen.

Die Polymerisation erfolgt üblicherweise bei Temperaturen von 50 bis 140°C und bei Normaldruck oder unter Eigendruck.

Als Initiatoren für die radikalische Polymerisation können die hierfür üblichen wasserlöslichen und wasserunlöslichen Peroxound/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, tert.-Butylhydroperoxid, Azo-bis-isobutyronitril, Azobis-(2-amidinopropan)dihydrochlorid oder 2,2'-Azo-bis-(2-methylbutyronitril). Geeignet sind auch Initiatormischungen oder Redox-Initiator-Systeme wie Ascorbinsäure/Eisen(II)sulfat/Natriumperoxodisulfat, tert.-Butylhydroperoxid/Natriumsulfit, tert.-Butylhydroperoxid/Natriumhydroxymethansulfinat. Die Initiatoren können in den üblichen Mengen eingesetzt werden, beispielsweise in Mengen von 0,05 bis 5 Gew.-%, bezogen auf die Menge der zu polymerisierenden Monomeren.

Die Kosmetikpräparate enthalten bevorzugt 0,1 bis 30 Gew.-% besonders bervorzugt 0,2 bis 20 Gew.-% des Polymers, bezogen auf das Präparat.

Ein weiterer Gegenstand der Erfindung ist ein Verfahren zur Herstellung der oben beschriebenen Polymere, bei dem nach der Polymerisation, die bevorzugt als Lösungspolymerisation ausgeführt wird, das so erhaltene Polymerisat direkt mit Wasserstoffperoxid und/oder Aktivkohle und/oder einem flüchtigen pflanzlichen Öl (ätherischen Öl) bei einer Temperatur zwischen 70 und 120°C behandelt wird.

Wasserstoffperoxid wird dabei in einer Menge bis zu 1 Gew.-%, Aktivkohle bis zu 5 Gew.-% und das ätherische Öl bis zu 1 Gew.-%, jeweils bezogen auf die Polymerlösung, eingesetzt.

Geeignete ätherische Öle sind beispielsweise Lavendel-, Rosen-, Zimt-, Mandel- und Kokosöl.

Durch dieses Verfahren erreicht man eine Verbesserung des Geruchs und auch der Farbe des Polymers.

Die Polymeren in den Kosmetikpräparaten werden bevorzugt entweder mit einer einwertigen Säure oder mit einer mehrwertigen Säure oder einer Polycarbonsäure, teil- oder neutralisiert, oder mit einem Quaternisierungsmittel (Alkylhalogenid oder Dialkylsulfat) in üblicher Weise quaternisiert.

Das Polymerisat wird insbesondere mit Phosphorsäure oder Säuregemisch mit Phosphorsäure teil- oder vollständig neutralisiert.

Als Säuren können organische Säuren, Mono-, Di-, Tricarbonsäuren (z.B. Fettsäure, Milchsäure, Weinsäure, Citronensäure) oder mineralische Säuren (z.B. Salzsäure, H₂SO₄, H₃PO₄) eingesetzt werden.

Um den Rest-Vinyllactam(VP oder VCap)-Gehalt niedrig zu erhalten, wird die Produktlösung nach der Polymerisation mit 2,5-Dimethyl-2,5-di(tert.-butylperoxy)hexan (Trigonox 101) bei ca. 130°C/3-10 h nachpolymerisiert. Damit wird der Restgehalt an Vinyllactam-Monomeren auf < 100 ppm gesenkt.

Die Erfindung betrifft auch die Verwendung der erfindungsgemäßen Polymerisate für kosmetische Zubereitung, insbesondere als Festigerpolymer in Haarspray, Schaumfestiger, Haarmousse, Haargel und Shampoos.

Weitere geeignete Anwendungsgebiete sind hautkosmetische Präparate und Nagellacke.

Die erfindungsgemäßen Polymerisate weisen bevorzugt eine Glastemperatur von über 25°C und einen K-Wert von 25 bis 70, vorzugsweise 35 bis 50 (gemessen nach Fikentscher) auf.

Ein weiterer Gegenstand der Erfindung sind Kosmetikpräparate, die das erfindungsgemäße Polymer in einer Menge von 0,1 bis 30 Gew.-%, bezogen auf das Präparat, enthalten.

Die Erfindung betrifft außerdem Haarkosmetikpräparate, enthaltend
(a) 0,2 bis 20 Gew.-% eines Polymers nach einem der Ansprüche 1 bis 6.
(b) 0 bis 10 Gew.-% eines konventionellen Haarfestigerpolymers,
(c) 0 bis 1 Gew.-% einer wasserdispergierbaren siloxanhaltigen Verbindung.
(d) 30 bis 99,5 Gew.-% eines Lösungsmittels oder Lösungsmittelgemisches aus Alkohol, insbesondere Ethanol, und Wasser.
(e) 0 bis 60 Gew.-% eines Treibmittels aus Dimethylether und/oder Propan/Butan.
(f) 0 bis 0,3 Gew.-% eines kosmetisch geeigneten Zusatzstoffes.

Man kann die erfindungsgemäß zu verwendenden Polymere auch mit herkömmlichen Haarkosmetik-Polymeren abmischen, falls spezielle Eigenschaften eingestellt werden sollen. Als herkömmliche Haarkosmetik-Polymere eignen sich beispielsweise
- kationische (quaternisierte) Polymere, z.B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z.B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nicht-ionische, wasserlösliche bzw. wasserdispergierbare Polymere oder Oligomere, wie Polyvinylcaprolactam, z.B. Luviskol Plus (BASF), oder Polyvinylpyrrolidon und deren Copolymere, insbesondere mit Vinylestern, wie Vinylacetat, z.B. Luviskol VA 37 (BASF); Polyamide, z.B. auf Basis von Itaconsäure und aliphatischen Diaminen;
- amphotere oder zwitterionische Polymere, wie die unter den Bezeichnungen Amphomor® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind;
- nichtionische, siloxanhaltige, wasserlösliche oder - dispergierbare Polymere, z.B. Polyethersiloxane, wie Tegopren® (Fa. Goldschmidt) oder Belsil® (Fa. Wacker).

Die erfindungsgemäß zu verwendenden Polymere eignen sich allgemein als Haarfestigerpolymer mit Konditionierungswirkung in kosmetischen Zubereitungen, vor allem in haarkosmetischen Zubereitungen wie Haarkuren, Haarlotionen, Haarspülungen, Haaremulsionen, Spitzenfluids, Egalisierungsmitteln für Dauerwellen, Hot-Oil-Treatment'-Präparate, Conditioner, Shampoos, Festigerlotionen oder Haarsprays.

Je nach Anwendungsgebiet können die haarkosmetischen Zubereitungen als Spray, Schaum, Gel, Gelspray oder Mousse appliziert werden. Bevorzugt ist die Verwendung als Haarspray.

Die haarkosmetischen Zubereitungen können neben den erfindungsgemäßen Polymeren und geeigneten Lösungsmitteln wie Wasser oder Wasser/Alkohol-Gemischen noch in der Kosmetik übliche Zusätze wie Emulgatoren, Konservierungsmittel, Parfümöle, Pflegestoffe wie panthenol, Collagen, Vitamine, Eiweißhydrolysate, Stabilisatoren, pH-Wert-Regulatoren, Farbstoffe und weitere übliche Additive enthalten.

Ein besonders geeigneter Zusatz für die erfindungsgemäßen Polymere sind Fettsäureamide, insbesondere solche mit einer Kettenlänge von C₁₂-C₂₂. Besonders bevorzugt sind Erucasäureamid und Stearinsäureamid. Die Fettsäureamide werden in einer Menge von bis zu 0,1 Gew.-%, bezogen auf das Polymer, eingesetzt.

Die erfindungsgemäßen Polymere können auch in kosmetischen Präparaten zum Schutz und zur Pflege der Haut, beispielsweise als Feuchtigkeitscreme oder -lotion, eingesetzt werden.

Allgemeine Herstellungsvorschrift (Lösungspolymerisation)

### Beispiel 1

- Zulauf 1:: 1200 g Monomergemisch aus 612 g tert.-Butylacrylat, 120 g Dimethylaminopropylacrylamid und 468 g Vinylpyrrolidon
- Zulauf 2:: 2,4 g Wako V59 = 2,2'-Azobis(2-methylbutyronitril) in 450 g Ethanol
- Zulauf 3:: 3,6 g Wako V59 in 90 g Ethanol
- Zulauf 4:: 77 g 40 %ig (in Ethanol) Phosphorsäure

Eine Mischung aus 240 g Zulauf 1, 60 g Zulauf 2 und 270 g Ethanol wurde auf 75°C erwärmt. Nach dem Anspringen der Polymerisation, erkennbar an einer Viskositätserhöhung, wurde der Rest des Zulaufs 1 in 4 h und Zulaufs 2 in 5 h bei 78°C unter Rühren zugegeben und noch 4 g bei 80°C polymerisiert. Nach Zudosierung von Zulauf 3 in 1/2 h wurde das Produkt 8 g/80°C nachpolymerisiert. Bei einer Temperatur von etwa 40°C unter Rühren wurde Zulauf 4 in ca. 30 min zudosiert. Man erhielt eine klare hellgelbe Lösung.

Analog können die anderen Produkte der folgenden Tabelle hergestellt werden. Die Polymere haben einen K-Wert (gemessen in 1 %iger ethanolischer Lösung) von 37 bis etwa 45 und eine Pro/Bu-Verträglichkeit > 60 %.

Die nachfolgende Tabelle zeigt die Werte für die Zusammensetzung (Gew.-%), die Curl-Retention, die Festigung, das "Flaking" und die Filmeigenschaften (die Klebrigkeit, die Auswaschbarkeit und den Griff).

### Beispiele

| | TBA | TMBA | NtBAM | SMA | DMAPMA | VP | VCap | Curl *) Retention [%] | Festigung *) | Fla-king *) | Film *) Klebrig./ Auswasch/ Griff |
|---|---|---|---|---|---|---|---|---|---|---|---|
| 1 | 51 | -- | -- | -- | 10 | 39 | -- | 75 | 2 | 2 | 2/2/1-2 |
| 2 | 55 | -- | -- | -- | 15 | 30 | -- | 79 | 1-2 | 1-2 | 1-2/1-2/1-2 |
| 3 | 40 | 15 | -- | -- | 16 | 29 | -- | 83 | 1-2 | 2 | 1-2/2/2 |
| 4 | 40 | -- | 15 | .. | 16 | 29 | -- | 87 | 1-2 | 2 | 1-2/2/1-2 |
| 5 | 45 | -- | -- | 10 | 16 | 29 | -- | 83 | 1-2 | 1 | 2/2/1 |
| 6 | 55 | -- | -- | -- | 17 | 28 | -- | 87 | 1-2 | 1 | 1-2/1-2/1 |
| 7 | 59 | -- | -- | -- | 23 | 18 | -- | 71 | 1-2 | 1-2 | 2/1/1-2 |
| 8 | 63 | -- | -- | -- | 30 | 7 | -- | 76 | 2 | 2 | 2-3/1/1-2 |
| 9 | 60 | -- | -- | -- | 30 | -- | 10 | 80 | 1-2 | 2 | 1-2/1-2/1-2 |
| 10 | 35 | -- | -- | 20 | 15 | 30 | -- | 67 | 2 | 1-2 | 2/1/2 |
| 11 | 25 | -- | -- | 30 | 15 | 30 | -- | 60 | 2-3 | 1 | 2-3/1/1-2 |
| 12 | -- | -- | 25 | 30 | 15 | 30 | -- | 77 | 1-2 | 1 | 1-2/1/2 |
| 13 | -- | -- | 15 | 35 | 15 | -- | 30 | 75 | 1-2 | 1-2 | 1-2/1/1-2 |

| | | | | | | | | | | | |
|---|---|---|---|---|---|---|---|---|---|---|---|
| *) Curl retention, Festigung, Flaking und Film wurden von der VOC 95-aerosol Haarspray-Formulierung (mit 50 Tl. Pro/Bu.) ermittelt TBA:tert.-Butylacrylat VP: Vinylpyrrolidon TMBA: tert.-Butylmethacrylat VCap:Vinylcaprolactam NtBAM: tert.-Butylacrylamid DMAPMA: Dimethylaminopropylmethacrylamid SMA: Stearinmethacrylat | | | | | | | | | | | |

Die Curl-Retention ist ein Maß für die haarfestigende Wirkung unter extremen klimatischen Bedingungen. Sie wird im Modellversuch an Haarlocken gemessen, die durch eine übliche Wasserwelle an ca. 15 cm langen Haaren erzeugt und mit der jeweiligen Sprayzubereitung aus 10 cm Entfernung 4 sec lang besprüht worden sind. Nach einer Verweilzeit von 5 h der aufgehängten Locken in einer Klimakammer bei 25°C und 90 % relativer Luftfeuchtigkeit wird die relative Verformung (Aufweitung) der Locken, bezogen auf ihre ursprüngliche Form, bestimmt. Ein hoher Wert bedeutet eine hohe Festigungswirkung, d.h. bei 100 % bliebe die ursprüngliche Form vollständig erhalten.

Die Festigungstest (die Stärke der Festigung eines Polymers die Haare am Modellkopf), der Flaking (Restpolymer am Haar nach dem Ausbürsten), die Klebrigkeit, die Auswaschbarkeit des Polymers aus dem Haar und der Griff (das Anfühlen des behandelten Haares) wurden jeweils mit Noten von 1 bis 4 von Fachleuten beurteilt; dabei bedeutet:
1 = sehr gut
2 = gut
3 = noch gut
4 = schlecht

### Anwendungstechnische Beispiele

• Aerosole Haarsprays:

| | | |
|---|---|---|
| VOC 95 = | Polymer Nr. 1 bis 9 | 5 Tl. |
| | Ethanol | 55 Tl. |
| | Pro/Bu | 40 Tl. |
| | Zusatz: Silikon, Parfum | |
| VOC 95 = | Polymer Nr. 1 bis 9 | 5 Tl. |
| | Ethanol | 45 Tl. |
| | Pro/Bu | 50 Tl. |
| | Zusatz: Silikon, Parfum | |
| VOC 80 = | Polymer Nr. 1 bis 9 | 5 Tl. |
| | Wasser | 15 Tl. |
| | Ethanol | 40 Tl. |
| | DME | 40 Tl. |
| | Zusatz: Silikon, Parfum | |
| VOC 55 | = Polymer Nr. 1 bis 9 | 3 Tl. |
| | Wasser | 42 Tl. |
| | Ethanol | 15 Tl. |
| | DME | 40 Tl. |
| | Zusatz: Silikon, Parfum | |

• Pumpsprays:

| | | |
|---|---|---|
| VOC 95 = | Polymer Nr. 1 bis 9 | 5 Tl. |
| | Ethanol | 95 Tl. |
| | Zusatz: Silikon, Parfum | |
| VOC 80 = | Polymer Nr. 1 bis 9 | 5 T1. |
| | Wasser | 15 Tl. |
| | Ethanol | 80 T1. |
| | Zusatz: Silikon, Parfum | |

| | |
|---|---|
| Polymer 1 bis 13 (25%ige wäßrige Lösung) | 20,0 |
| Cremophor A 25 (Ceteareth 25 / BASF) | 0,2 |
| Comperlan KD (Coamide DEA / Henkel) | 0,1 |
| Wasser | 69,7 |
| Propan/Butan | 10,0 |
| Erucasäureamid | |
| 0,0005 | |
| Weitere Zusätze: Parfüm, Konservierungsmittel | ... |

### Herstellung: Einwiegen und unter Rühren lösen. Abfüllen und Treibgas zusetzen.

| Conditioner Shampoo | | [%] |
|---|---|---|
| | | |
| A) | Texapon NSO 28%ig (Sodium Laureth Sulphate / Henkel) | 50,0 |
| | Comperlan KD (Coamide DEA / Henkel) | 1,0 |
| | Polymer 1 bis 13 (25%ige wäßrige Lösung) | 20,0 |
| | Erucasäureamid | 0,001 |
| | q.s. Parfümöl | |
| | | |
| B) | Wasser | 27,5 |
| | Natriumchlorid | 1,5 |
| | q.s. Konservierungsmittel ... | |

### Herstellung: Einwiegen und unter Rühren Phasen A und B getrennt lösen und mischen. Phase B langsam in Phase A einrühren.

| Standard O/W-Creme | | |
|---|---|---|
| | | |
| Ölphase | [%) | CTFA Name |
| | | |
| Cremophor A6 | 3,5 | Ceteareth-6 (and) Sterayl Alkohol |
| Cremophor A25 | 3,5 | Ceteareth-25 |
| Glycerinmonostearat s.e. | 2,5 | Glyceryl Stearate |
| Paraffinöl | 7,5 | Paraffin Oil |
| Cetylalkohol | 2,5 | Cetyl Alkohol |
| Luvitol EHO | 3,2 | Cetearyl Octanoate |
| Vitamin-E-acetate | 1,0 | Tocopheryl Acetate |
| Stearinsäureamid | 0,001 | |
| Nip-Nip | 0,1 | Methyl- und Propyl-4-hydroxybenzoate (7:3) |
| Standard O/W-Creme | | |
| | | |
| Wasserphase | [%] | |
| | | |
| Polymer 1 bis 13 | 1,5 | |
| Wasser | 73,6 | Water |
| 1,2-Propylenglykol | 1,0 | Propylene Glycol |
| Germall II | 0,1 | Imidazolidinyl-Urea |

### Herstellung: Einwiegen und unter Rühren die Öl-Phasen und Wasser-Phase getrennt bei einer Temperatur von ca. 80°C homogenisieren. Wasser-Phase langsam in Öl-Phase einrühren. Unter Rühren langsam auf RT abkühlen.

| O/W-Lotion | | |
|---|---|---|
| Ölphase | [%] | CTFA Name |
| | | |
| Cremophor A6 | 2,0 | Ceteareth-6 (and) Sterayl Alkohol |
| Cremophor A25 | 2,0 | Ceteareth-25 |
| Glycerinmonostearat | 6,0 | Glyceryl Stearate |
| Paraffinöl | 0,9 | Paraffin Oil |
| Tegiloxan 100 | 0,1 | Dimethicone |
| Cetylalkohol | 1,5 | Cetyl Alkohol |
| Luvitol EHO | 12,0 | Cetearyl octanoate |
| Vitamin-E-acetate | 0,4 | Tocopheryl Acetate |
| Erucasäureamid | 0,001 | |
| Nip-Nip | 0,1 | Methyl- und Propyl-4-hydroxybenzoate (7:3) |
| | | |
| Wasserphase | [%] | |
| | | |
| Polymer 1 bis 13 | 1,0 | |
| Wasser | 73,4 | Water |
| 1,2-Propylenglykol | 1,0 | Propylene Glycol |
| Germall II | 0,1 | Imidazolidinyl-Urea |

### Herstellung: Einwiegen und unter Rühren die Öl-Phasen und Wasser-Phase getrennt bei einer Temperatur von ca. 80°C homogenisieren. Wasser-Phase langsam in Öl-Phase einrühren. Unter Rühren langsam auf RT abkühlen.

### Beispiel 14 (TBA:DMAPMA:VP:SMA:Balsil = 50,5:15:28:6:0,5)

(1) 2,4 g Belsil® DMC 6031 (Wacker), 6 g Wasser und 64 g Ethanol wurden in den mit N₂ vorgespülten Polymerisationskessel eingefüllt.
(2) Die Lösung wurde auf 75°C erwärmt und gerührt.
(3) 242,4 g TBA, 72 g DMAPMA, 134,3 g VP und 28,8 g SMA wurden zugefügt.
(4) 1 g 2,2'-Azobis(2-methylbutyronitril) (V-59, WAKO), aufgelöst in 170 g Ethanol wurden zugegeben.
(5) Nachdem eine Temperatur von 70°C erreicht worden ist, wurden 96 g des Monomerzulaufs und 17 g der Initiatorlösung zugegeben.
(6) Der restliche Monomerzulauf und die Initiacorzugabe wurden innerhalb von 4 bis 5 Stunden zugegeben.
(7) Nach Beendigung des Zulaufs wurde noch 2 Stunden bei 80°C gerührt.
(8) Gegebenenfalls nach Behandlung mit Wasserstoffperoxid und Aktivkohle wurde das Polymerisat mit Milchsäure neutralisiert.

Analog zu Beispiel 14 wurden die folgenden Polymere (Beispiel 15 bis 22) hergestellt.

**Tabelle**

| Bsp. | TBA | DMAPMA | VP | SMA | DMC 6031 |
|---|---|---|---|---|---|
| 15 | 29 | 15 | 25 | 29 | 2 |
| 16 | 36 | 16 | 28 | 18 | 2 |
| 17 | 47 | 16 | 28 | 0 | 9 |
| 18 | 54 | 16 | 28 | 0 | 2 |
| 19 | 50 | 15 | 28 | 6 | 1 |
| 20 | 56 | 15 | 28 | 0 | 1 |
| 21 | 50 | 16 | 28 | 6 | 0 |
| 22 | 50,5 | 15 | 28 | 6 | 0,5 |

## Patentansprüche

1. Kationische Polymerisate, erhältlich durch radikalische Copolymerisation von
(a) 50 bis 70 Gew.-% eines oder mehrerer Monomeren der Formel I 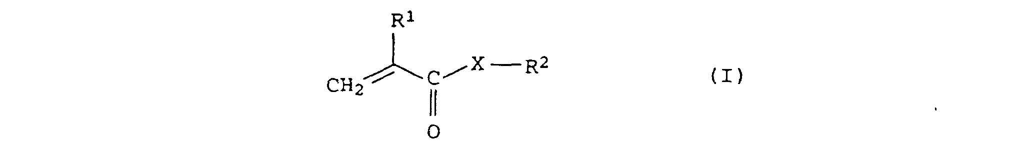
X = O, NR¹,
R¹ = H, C₁-C₈-Alkyl,
R² = tert.-Butyl,
(b) 5 bis 45 Gew.-% eines oder mehrerer Monomeren der Formel II 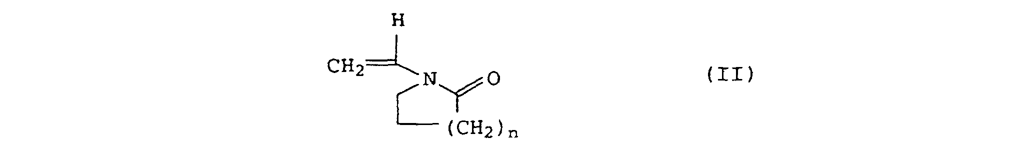 mit n = 1 bis 3,
(c) 5 bis 40 Gew.-% eines monoethylenisch ungesättigten Monomers mit mindestens einer aminhaltigen Gruppe,
(d) 0 bis 5 Gew.-% eines polyalkylenoxid-haltigen Silikonderivats,
wobei bis zu 40 Gew.-%, bezogen auf (a), (b), (c) und (d) des Monomeren (a) durch ein Monomer der Formel I mit R² = C₂-C₂₂-Alkyl ersetzt sein können und wobei bis zu 4 Gew.-%, bezogen auf die gesamten (a) + (b) + (c), des Monomeren (b) durch ein hydrophiles nichtionisches Monomer der Struktur III ersetzt sein kann 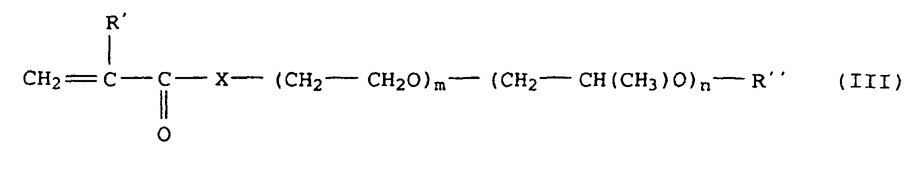
R' = H, CH₃
X = O, NH
m = 0 bis 50
n = 0 bis 50
m + n ≥ 5
R'' = H, C₁-C₂₂-Alkyl oder Phenyl.

2. Polymerisate nach Anspruch 1, **dadurch gekennzeichnet, daß** sie erhältlich sind durch radikalische Copolymerisation von
(a) 51 bis 65 Gew.-% des Monomeren der Formel I,
(b) 7 bis 39 Gew.-% des Monomeren der Formel II,
(c) 10 bis 30 Gew.-% des aminhaltigen Monomeren.

3. Polymerisate nach Anspruch 1, **dadurch gekennzeichnet, daß** das Monomer (a) tert.-Butylacrylat, N-tert.-Butyl-acrylamid und/oder tert.-Butylmethacrylat bedeutet.

4. Polymerisate nach Anspruch 1, **dadurch gekennzeichnet, daß** das Monomer (b) Vinylpyrrolidon und/oder Vinylcaprolactam ist.

5. Polymerisate nach Anspruch 1, **dadurch gekennzeichnet, daß** das Monomer (c) Dimethylaminoalkyl-(meth)acrylat und/oder Dimethylaminoalkyl-(meth)acrylamid ist.

6. Polymerisate nach Anspruch 1, **dadurch gekennzeichnet, daß** es sich bei den Monomeren der Formel I mit R² = C₂-C₂₂-Alkyl um N-Butylacrylamid, N-Octylacrylamid, Lauryl(meth)acrylat oder Stearyl(meth)-acrylat handelt.

7. Verwendung von Polymerisaten nach Anspruch 1 bis 6 für kosmetische Zubereitungen.

8. Verwendung nach Anspruch 7 als Festigerpolymer in Haarspray, Schaumfestiger, Haarmousse, Haargel oder Shampoos.

9. Kosmetikpräparate, enthaltend ein Polymer nach Anspruch 1 in einer Menge von 0,1 bis 30 Gew.-% bezogen auf das Präparat.

10. Kosmetikpräparate nach Anspruch 9, **dadurch gekennzeichnet, daß** das Polymer mit einer einwertigen Säure, bevorzugt mit einer mehrwertigen Säure oder einer Polycarbonsäure, teiloder neutralisiert wird, oder mit einem Quaternisierungsmittel quaternisiert wird.

11. Kosmetikpräparate nach Anspruch 10, **dadurch gekennzeichnet, daß** das Polymer mit Phosphorsäure oder einem Säuregemisch mit Phosphorsäure teil- oder vollständig neutralisiert wird.

12. Verwendung von Polymerisaten nach einem der Ansprüche 1 bis 6 mit einer Glastemperatur > 25°C und einem K-Wert von 25 bis 70, vorzugsweise 35 bis 50, für die Haarkosmetik.

13. Haarkosmetikpräparate, enthaltend
(a) 0,2 bis 20 Gew.-% eines Polymers nach einem der Ansprüche 1 bis 6.
(b) 0 bis 10 Gew.-% eines konventionellen Haarfestigerpolymers,
(c) 0 bis 1 Gew.-% einer wasserdispergierbaren siloxanhaltigen Verbindung.
(d) 30 bis 99,5 Gew.-% eines Lösungsmittels oder Lösungsmittelgemisches aus Alkohol und Wasser.
(e) 0 bis 60 Gew.-% eines Treibmittels aus Dimethylether und/oder Propan/Butan.
(f) 0 bis 0,3 Gew.-% eines kosmetisch geeigneten Zusatzstoffes.

14. Verwendung nach Anspruch 7 als Bestandteil in hautkosmetischen Präparaten.

15. Verwendung nach Anspruch 14, **dadurch gekennzeichnet, daß** zusätzlich ein Fettsäureamid eingesetzt wird.

## Claims

1. A cationic polymer obtainable by free-radical copolymerization of
(a) from 50 to 70% by weight of one or more monomers of the formula I 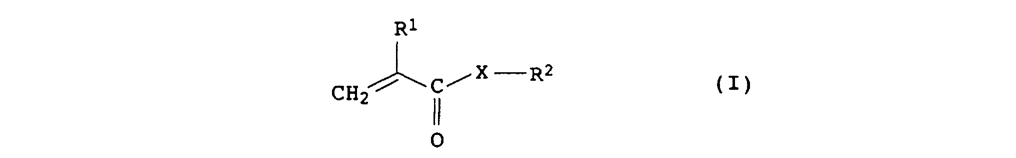
X = O, NR¹,
R¹ = H, C₁-C₈-alkyl,
R² = tert-butyl,
(b) from 5 to 45% by weight of one or more monomers of the formula II 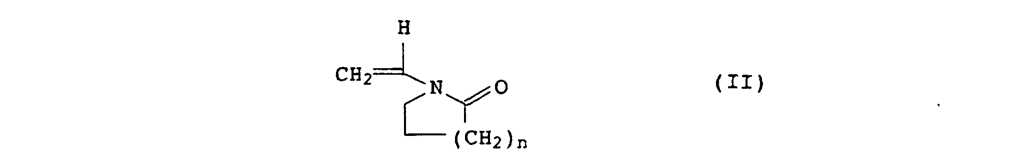 where n = 1 to 3,
(c) from 5 to 40% by weight of a monoethylenically unsaturated monomer having at least one amine-containing group,
(d) from 0 to 5% by weight of a polyalkylene oxide-containing silicone derivative,
where up to 40% by weight, based on (a), (b), (c) and (d), of the monomer (a) can be replaced by a monomer of the formula I where R² = C₂-C₂₂-alkyl, and where up to 4% by weight, based on the total (a) + (b) + (c), of monomer (b) can be replaced by a hydrophilic nonionic monomer of the structure III 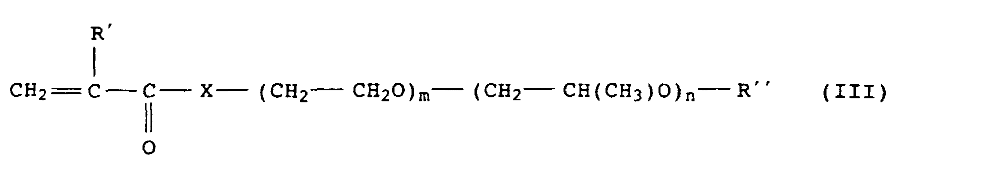
R' = H, CH₃
X = O, NH
m = from 0 to 50
n = from 0 to 50
m + n ≥ 5
R'' = H, C₁-C₂₂-alkyl or phenyl.

2. A polymer as claimed in claim 1, obtainable by free-radical copolymerization of
(a) from 51 to 65% by weight of the monomer of the formula I,
(b) from 7 to 39% by weight of the monomer of the formula II,
(c) from 10 to 30% by weight of the amine-containing monomer.

3. A polymer as claimed in claim 1, wherein the monomer (a) is tert-butyl acrylate, N-tert-butylacrylamide and/or tert-butyl methacrylate.

4. A polymer as claimed in claim 1, wherein the monomer (b) is vinylpyrrolidone and/or vinylcaprolactam.

5. A polymer as claimed in claim 1, wherein the monomer (c) is dimethylaminoalkyl (meth)acrylate and/or dimethylaminoalkyl (meth)acrylamide.

6. A polymer as claimed in claim 1, wherein the monomers of the formula I where R² = C₂-C₂₂-alkyl are N-butylacrylamide, N-octylacrylamide, lauryl (meth)acrylate or stearyl (meth)acrylate.

7. The use of polymers as claimed in claim 1 to 6 for cosmetic preparations.

8. The use as claimed in claim 7 as setting polymers in hair spray, foam setting compositions, hair mousse, hair gel or shampoos.

9. A cosmetic preparation comprising a polymer as claimed in claim 1 in an amount of from 0.1 to 30% by weight, based on the preparation

10. A cosmetic preparation as claimed in claim 9, wherein the polymer is partially or completely neutralized using a monohydric acid, preferably using a polyhydric acid or a polycarboxylic acid, or is quaternized using a quaternizing agent.

11. A cosmetic preparation as claimed in claim 10, wherein the polymer is partially or completely neutralized using phosphoric acid or an acid mixture containing phosphoric acid.

12. The use of polymers as claimed in one of claims 1 to 6 having a glass transition temperature of > 25°C and a K value of from 25 to 70, preferably from 35 to 50, for hair cosmetics.

13. A hair cosmetic preparation comprising
(a) from 0.2 to 20% by weight of a polymer as claimed in one of claims 1 to 6,
(b) from 0 to 10% by weight of a conventional hair-setting polymer,
(c) from 0 to 1% by weight of a water-dispersible siloxane-containing compound,
(d) from 30 to 99.5% by weight of a solvent or solvent mixture of alcohol and water,
(e) from 0 to 60% by weight of a propellant comprising dimethyl ether and/or propane/butane, and
(f) from 0 to 0.3% by weight of a cosmetically suitable additive.

14. The use as claimed in claim 7 as a constituent in cosmetic skin preparations.

15. The use as claimed in claim 14, wherein a fatty acid amide is additionally used.

## Revendications

1. Polymères cationiques, pouvant être obtenus par copolymérisation radicalaire de
(a) 50 à 70 % en poids d'un ou plusieurs monomères de formule I 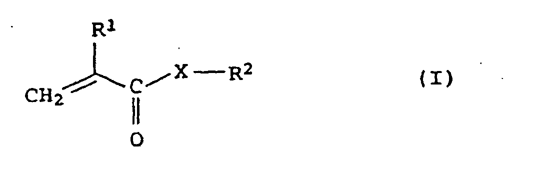 où
X = O, NR¹,
R¹ = H, alkyle en C₁-C₈,
R² = tert-butyle,
(b) 5 à 45 % en poids d'un ou plusieurs monomères de formule II 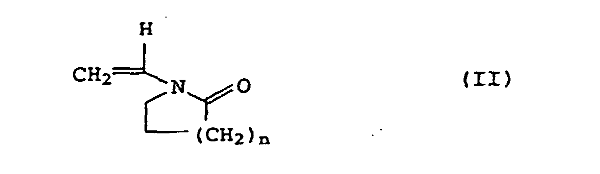 avec n = 1 à 3,
(c) 5 à 40 % en poids d'un monomère à insaturation monoéthylénique ayant au moins un groupe contenant une amine,
(d) 0 à 5 % en poids d'un dérivé de silicium contenant du poly(oxyde d'alkylène),
tandis que jusqu'à 40 % en poids, par rapport à (a), (b), (c) et (d) du monomère (a) peuvent être remplacés par un monomère de formule I avec R² = alkyle en C₂-C₂₂ et tandis que jusqu'à 4 % en poids, par rapport à l'ensemble (a) + (b) + (c), du monomère (b) peut être remplacé par un monomère non-ionique hydrophile de structure III 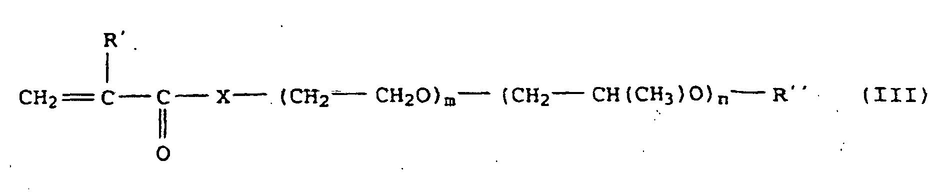 où
R' = H, CH₃
X = O, NH
m = 0 à 50
n = 0 à 50
m+n ≥ 5
R" = H, alkyle en C₁-C₂₂ou phényle.

2. Polymères selon la revendication 1, **caractérisés par le fait qu'**ils peuvent être obtenus par copolymérisation radicalaire de
(a) 51 à 65 % en poids du monomère de formule I,
(b) 7 à 39 % en poids du monomère de formule II,
(c) 10 à 30 % en poids du monomère contenant une amine.

3. Polymères selon la revendication 1, **caractérisés par le fait que** le monomère (a) est l'acrylate de tert-butyle, le N-tert-butyl-acrylamide et/ou le méthacrylate de tert-butyle.

4. Polymères selon la revendication 1, **caractérisés par le fait que** le monomère (b) est la vinylpyrrolidone et/ou le vinylcaprolactame.

5. Polymères selon la revendication 1, **caractérisés par le fait que** le monomère (c) est le (méth)acrylate de diméthylaminoalkyle et/ou le diméthylaminoalkyl-(méth)acrylamide.

6. Polymères selon la revendication 1, **caractérisés par le fait qu'**il s'agit en ce qui concerne les monomères de formule I avec R² = alkyle en C₂-C₂₂ de N-butylacrylamide, de N-octylacrylamide, de (méth)acrylate de lauryle ou de (méth)acrylate de stéaryle.

7. Utilisation de polymères selon la revendication 1 à 6 pour des préparations cosmétiques.

8. Utilisation selon la revendication 7 comme polymère renforçateur dans des sprays capillaires, renforçateurs de mousse, mousses capillaires, gel capillaires ou shampooings.

9. Préparations cosmétiques, contenant un polymère selon la revendication 1 en une quantité de 0,1 à 30 % en poids par rapport à la préparation.

10. Préparations cosmétiques selon la revendication 9, **caractérisées par le fait que** le polymère est partiellement ou totalement neutralisé avec un acide monovalent, de préférence avec un acide plurivalent ou un polyacide carboxylique, ou est quaternisé avec un agent de quaternisation.

11. Préparations cosmétiques selon la revendication 10, **caractérisées par le fait que** le polymère est partiellement ou totalement neutralisé avec de l'acide phosphorique ou un mélange d'acides avec de l'acide phosphorique.

12. Utilisation de polymères selon l'une des revendications 1 à 6 ayant une température de transition vitreuse > 25°C et un indice K de 25 à 70, de préférence de 35 à 50, pour la cosmétique capillaire.

13. Préparations de cosmétique capillaire, contenant
(a) 0,2 à 20 % en poids d'un polymère selon l'une des revendications 1 à 6,
(b) 0 à 10 % en poids d'un polymère conventionnel renforçateur des cheveux,
(c) 0 à 1 % en poids d'un composé hydrodispersable contenant du siloxane,
(d) 30 à 99,5 % en poids d'un solvant ou d'un mélange de solvants comprenant de l'alcool et de l'eau,
(e) 0 à 60 % en poids d'un agent propulseur, comprenant de l'éther diméthylique et/ ou du propane/butane,
(f) 0 à 0,3 % en poids d'un additif approprié en cosmétique.

14. Utilisation selon la revendication 7 comme composant dans des préparations cosmétiques pour la peau.

15. Utilisation selon la revendication 14, **caractérisée par le fait qu'**on utilise en outre un amide d'acide gras.
